# EUROPEAN PATENT APPLICATION

(11) **EP 2 910 267 A1**
(43) Date of publication of application: **26.08.2015**
(21) Application number: 13848347.4
(22) Date of filing: 23.07.2013
(51) Int. Cl.: A61M 5/34, A61M 5/165, A61M 5/38

(54) **FILTER CAP FOR SYRINGE**

(30) Priority: 22.10.2012 KR 20120117088
(71) Applicant: Kim, Young Sang, Yongin-si, Gyeonggi-do 448-992 (KR); Kim, Young Hwan, Yongin-si, Gyeonggi-do 448-981 (KR)
(72) Inventor: Kim, Young Sang, Yongin-si, Gyeonggi-do 448-992 (KR); Kim, Young Hwan, Yongin-si, Gyeonggi-do 448-981 (KR)
(74) Representative: Garavelli, Paolo
(86) International application number: PCT/KR2013/006559
(87) International publication number: WO 2014/065489

(57) **Abstract**

The present invention relates to a filter cap for a syringe, and, more specifically, to one comprising: a housing having a through-passing injection-liquid inflow passage, a syringe needle insertion passage that passes through so as to link through to the injection-liquid inflow passage in such a way that a syringe needle can be inserted therein, and an injection needle tight-fitting passage that passes through so as to link through to the syringe needle insertion passage in such a way that it is possible to tightly fit the syringe needle therein and form a negative pressure; and a filter which is fitted by melt adhesion to a through-linking portion of the injection-liquid inflow passage and the syringe needle insertion passage in such a way that it is possible to filter the injection liquid.

## Description

### [Technical Field]

The present invention relates to a filter cap for a syringe, and more particularly, to a filter cap for a syringe capable of protecting a syringe needle by including a filter and a housing, preventing foreign substances from being injected into the human body by preventing foreign substances such as fine glass splinters generated and precipitated when an insertion-liquid glass ampoule is snapped off from being introduced into the syringe, filling the syringe with an insertion liquid without exposing the syringe needle to the outside, and preventing a negligent accident that may occur by being pricked by the syringe needle at the time of replacing the syringe needle with another syringe needle.

### [Background Art]

Since an insertion-liquid glass ampoule is used by snapping off the neck, fine glass splinters are mixed with an insertion liquid. A syringe is filled with the insertion liquid and the fine glass splinters, and thus, there is a problem in that the fine glass splinters are injected into the human body.

When the fine glass splinters generated when the glass ampoule is opened by snapping off the neck are injected into the human body through the syringe needle, since a thrombus is caused by the injected glass splinters, a possibility that and a fatal disease such as myocardial infraction or a stroke will be caused has been raised in the medical field.

According to the research report of Korean Food and Drug Administration, when the glass splinters mixed with the insertion liquid of the glass ampoule are injected into the body together with medication through intravenous injection, a possibility that a pathology change such as granuloma or inflammatory response of an organ such as lungs, a liver or a spleen will be caused has been raised. Particularly, according to the research report of Korean Food and Drug Administration in 2004, since the capillary vessel of the lungs of the human being has a diameter of 10 µm, when insolubility foreign substances such as glass splinters having a diameter greater than that of the capillary vessel are accumulated in the human body, it has been pointed out that a serious disease such as pulmonary embolism may be caused.

There are greatly two related arts for solving such problems.

The first related art is a syringe needle assembly including a filter attached therein (Korean Patent Publication No. 10-2012-0087587, hereinafter referred to as a filter needle), and the second related art is a filter cap (Korean Patent Publication No. 10-2005-0108169, U.S. Patent No. 4180071) capable of being attached to a general syringe needle assembly.

A procedure of using the filter needle by a user such as a nurse is performed in the following nine steps. A casing is removed, a safety cap is removed, an insertion liquid is taken in, the filter needle is removed, a safety cap of a general syringe needle is removed, the general syringe needle is coupled to a syringe, the insertion liquid is injected, the general syringe needle is separated, and the general syringe needle is discarded.

A procedure of using the filter cap by the user such as a nurse is performed in the following seven steps. A casing is removed, a safety cap is removed, an insertion liquid is taken in, the filter cap is removed, the insertion liquid is injected, the general syringe needle is separated, and the general syringe needle is discarded.

The filter cap is conveniently used in virtue of reduced steps by two steps. In addition, a possibility that the needle will be contaminated or a possibility that an accident that the human body is pricked will occur by the needle at the time of replacing the needle with another needle is considered, these procedures are not omitted, and thus, an accident occurrence possibility is eliminated.

In addition to such advantages for use, there is an advantage of reducing prime cost by using not two safety caps but one safety cap.

The filter cap type of the related art has the aforementioned advantage as compared to the filter needle type, but needs to be improved. The configuration and problems of the filter cap type of the related art are as follows.

In the filter cap of the related art, the filter is attached by being fixed to a penetrating portion within a housing by a protrusion, and a negative pressure is generated due to a portion that comes in close contact with a syringe-needle hub of a syringe needle assembly.

However, the filter cap of the related art is an insertion type in which the filter is fixed by the protrusion, and since the filter is not separated from the protrusion, the thickness of the filter is thick. Since the filter is thick, there is a problem in that large force is needed to taken in the insertion liquid. Since the filter attached therein is thick, the diameter of the penetrating portion is large, and thus, the outer diameter of the filter cap is large. As a result, since the hole of the ampoule and the outer diameter of the filter cap are similar to each other, there are problems in that it is difficult to insert the syringe needle into the ampoule and the syringe needle is contaminated. Moreover, since the volume of a sealed space within the housing is large by coming in close contact with the syringe needle hub, it is difficult to generate a negative pressure, and thus, it is difficult to taken in the insertion liquid.

In addition to such problems for use, since the filter is fixed to the inside by the protrusion, a manufacturing process is complicated. In order to attach the filter to the inside, there is a problem in that after the housing is manufactured as not one component but two or more components, the filter is attached to the housing, and the housing is assembled. There is a problem in that a separate process of forming the protrusion for fixing the filter to the housing is needed after the filter is inserted into the housing.

### [Disclosure]

### [Technical Problem]

In order to solve the aforementioned problems of the filter cap for a syringe of the related art, an object of the present is to provide a filter cap that has a filter thickness thinner than that of the related art, is easily inserted into the ampoule without contamination since the outer diameter of the ampoule insertion pipe is sufficiently smaller than the hole of the ampoule, and reduces manufacturing cost with high productivity in virtue of a simple manufacturing process.

Another object of the present invention is to provide a filter cap that easily fills a syringe with an insertion liquid in virtue of a small volume of a sealed space.

### [Technical Solution]

In order to achieve the aforementioned object, a filter cap according to the present invention includes a filter attached to the inside of a housing through melting. A syringe-needle tight-fitting passage is formed to come in close contact with a syringe needle in order to generate a negative pressure within the housing, an oil film is formed on the syringe-needle tight-fitting passage in order to further generate a negative pressure, and an inclined passage is formed within the housing so as to allow the filter cap to be gently fitted to the syringe needle.

### [Effect of the Invention]

Since the housing is formed as a single member and the filter is attached to the housing through melting, the filter cap of the present invention has an advantage of a simple manufacturing process.

Further, the filter attached to the housing through melting has an advantage of filling the syringe with the insertion liquid by taking in the insertion liquid in virtue of a thinner thickness than that of the related art.

Moreover, since the volume of a sealed space for forming a negative pressure is small by bringing the filter into close contact with the syringe needle, the filter cap has an advantage of easily filling the syringe with the insertion liquid by taking in the insertion liquid.

In addition, since the filter attached to the housing through melting has a smaller area than the filter cap of the related art, the outer diameter of the ampoule insertion pipe of the housing is small, and thus, there is an advantage that it is easy to insert the syringe into the ampoule and the contamination of the syringe is reduced.

### [Description of Drawings]

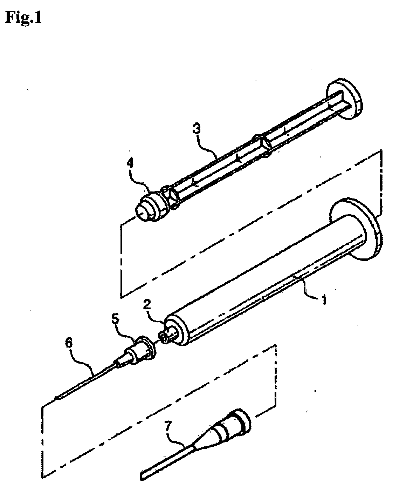

Fig. 1 is an exploded perspective view of a filter cap according to the present invention and a general disposable syringe.
Fig. 2 is a perspective view showing the filter cap according to the present invention.
Fig. 3 is a cross-sectional view showing the filter cap according to the present invention.
Fig. 4 is a front view showing a syringe needle assembly used for the general disposable syringe.
Fig. 5 is a cross-sectional view showing a connected state of the filter cap according to the present invention and the syringe needle assembly.
Fig. 6 is a diagram showing a utilization state where a syringe is filled with an insertion liquid of an insertion-liquid ampoule by using the filter cap according to the present invention.

### [Best Mode]

As the terms used in the present invention, general terms that are widely used at present are selected, but terms that are arbitrarily selected by the applicant are used in particular cases. In this case, these terms should be interpreted as not the titles of the terms but the meaning described in the detailed description for implementing the invention or the meaning of the terms.

Hereinafter, a technical configuration of the present invention will be described in detail with reference to preferred embodiments illustrated in the accompanying drawings.

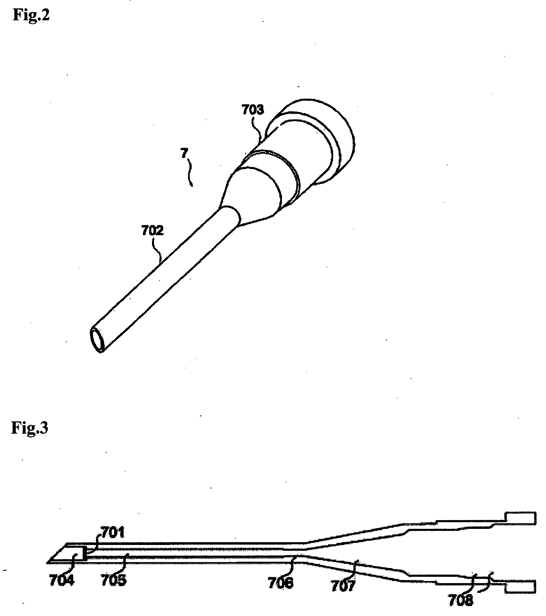

Fig. 2 is a perspective view showing a filter cap 7 according to the present invention, and Fig. 3 is a cross-sectional view showing the filter cap 7 according to the present invention.

Referring to Figs. 2 and 3, the filter cap 7 includes a filter 701, an ampoule insertion pipe 702, a disassemble support 703, an injection-liquid inflow passage 704, a syringe-needle insertion passage 705, a syringe-needle tight-fitting passage 706, an inclined passage 707, and a syringe-needle-fixing-hub insertion passage 708.

In this case, the filter 701 is fixed to a connection portion of the injection-liquid inflow passage 704 and the syringe-needle insertion passage 705 by being attached through melting. Since a diameter of a capillary vessel is 10 µm and it is of critical significance that a glass splinter having a diameter smaller than that of the capillary vessel is filtered, it is preferred that the filter 701 have a permeability of 10 µm or less. In the preferred embodiment of the present invention, a filter having a permeability of 5 µm or less is used.

Further, the filter 701 may be made of various materials that do not react with an injection liquid by virtue of chemical stability and can be attached through melting.

Meanwhile, the ampoule insertion pipe 702 is a portion that is inserted into an ampoule 8. Since an inner diameter of an unsealed portion of the unsealed ampoule 8 is generally from 4 mm to 10 mm, the ampoule insertion pipe needs to have an outer diameter of such a range or less. It is preferred that the ampoule insertion pipe have an outer diameter of 3 mm or less in order to prevent contact contamination.

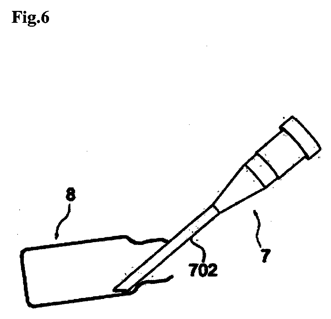

Meanwhile, the injection-liquid inflow passage 704 is a portion into which an injection liquid of the ampoule 8 is introduced. When a syringe is filled with the injection liquid as shown in Fig. 6, since one front end of the injection-liquid inflow passage on an external side of the housing into needs to come in close contact with the wall of the ampoule, it is convenient to form the injection-liquid inflow passage to be inclined.

In this case, in order to attach the filter 701 to the connection portion through melting, it is necessary to form a protrusion. Only when the diameter of the injection-liquid inflow passage 704 is greater than that of the syringe-needle insertion passage 705, since the protrusion is generated, the injection-liquid inflow passage 704 needs to have a diameter greater than that of the syringe-needle insertion passage. Thus, in the preferred embodiment of the present invention, a diameter difference of about 0.8 mm is generated.

Meanwhile, the syringe-needle insertion passage 705 is a portion into which a syringe needle 6 is inserted, and passes in the housing by being communicatively connected to the injection-liquid inflow passage 704. The syringe-needle insertion passage needs to have a diameter greater than an outer diameter of the syringe needle 6 to be inserted. However, as a difference between the diameter of the syringe-needle insertion passage and the outer diameter of the syringe needle 6 becomes higher, a negative pressure becomes smaller. Thus, such a difference between the diameter of the syringe-needle insertion passage and the outer diameter of the syringe needle may be set such that the syringe needle does not come in close contact with the syringe-needle insertion passage. In the preferred embodiment of the present invention, a gap of 0.275 mm is generated therebetween.

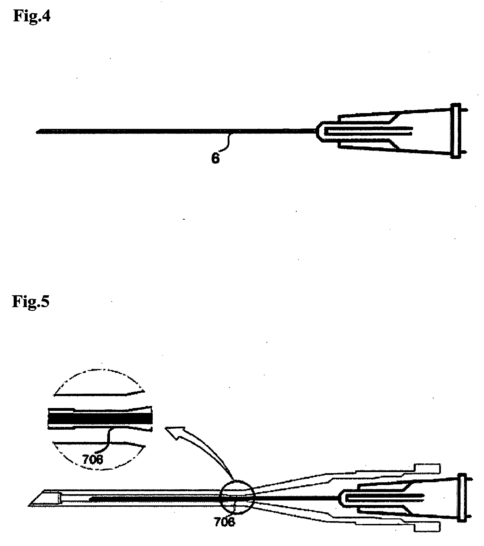

Meanwhile, the syringe-needle tight-fitting passage 706 is a portion that comes in close contact with the syringe needle when the syringe needle 6 is inserted, and passes in the housing by being communicatively connected to the syringe-needle insertion passage 705. Referring to Fig. 5, the syringe-needle tight-fitting passage needs to have a diameter capable of coming in close contact with the syringe needle 6 to be inserted, and needs to have a slight gap so as not to be fixed such that the filter cap can be detachably attached. In the preferred embodiment of the present invention, a gap of 0.075 mm is generated therebetween.

In this case, an oil film (not shown) is formed on the syringe-needle tight-fitting passage 706, and a negative pressure can be further generated by allowing the gap between the syringe-needle tight-fitting passage and the syringe needle to be filled with the oil film.

Meanwhile, the inclined passage 707 is a guide portion that allows the syringe needle 6 to be moved to the syringe-needle tight-fitting passage 706 without being stopped when the syringe needle 6 is inserted, and passes in the housing by being communicatively connected to the syringe-needle tight-fitting passage 706.

Meanwhile, the syringe-needle-fixing-hub insertion passage 708 is a portion into which the syringe-needle fixing hub 5 is inserted, passes in the housing by being communicatively connected to the inclined passage 707, and passes in the housing while being paired with the syringe-needle fixing hub 5 in order to insert the syringe-needle fixing hub 5.

Consequently, the filter cap 7 according to the embodiment of the present invention is convenient to use with the above-described configuration, and can reduce a contamination possibility or a needle accident possibility.

Furthermore, it is possible to reduce manufacturing costs with a simple configuration in which the filter is attached to an integrated housing through melting.

Moreover, since the filter cap can be manufactured using a small amount of materials, an excellent effect can be achieved in view of environmental protection.

As described above, while the present invention has been illustrated and described in conjunction with the preferred embodiment, the present invention is not limited to the aforementioned embodiment. The embodiment can be changed and modified in various forms by those skilled in the art without departing from the spirit of the invention.

## Claims

1. A filter cap (7) for a syringe comprising:
a housing in which an insertion-liquid inflow passage (704) passes, a syringe-needle insertion passage (705) communicatively connected to the insertion-liquid inflow passage (704) passes such that a syringe needle (6) is inserted, a syringe-needle tight-fitting passage (706) communicatively connected to the syringe-needle insertion passage (705) passes such that a negative pressure is generated by coming in contact with the syringe needle (6); and
a filter (701) that is attached to a connection portion of the insertion-liquid inflow passage (704) and the syringe-needle insertion passage (705) through melting such that an insertion liquid introduced into the insertion-liquid inflow passage (704) is filtered.

2. The filter cap (7) for a syringe according to claim 1,
wherein an oil film is formed on the syringe-needle tight-fitting passage (706) in order to bury a gap between the syringe needle (6) and the syringe-needle tight-fitting passage (706).

3. The filter cap (7) for a syringe according to claim 1 or 2,
wherein an inclined passage (707) communicatively connected to the syringe-needle tight-fitting passage (706) is formed such that the syringe needle (6) is inserted without being stopped.

4. The filter cap (7) for a syringe according to claim 1 or 2,
wherein one front end of the insertion-liquid inflow passage (704) on an external side of a housing is inclined such that the front end of the insertion-liquid inflow passage (704) on the external side the housing comes in close contact with a wall of an ampoule (8) at the time of taking in the ampoule (8).
